# EUROPEAN PATENT APPLICATION

(11) **EP 1 178 103 A1**
(43) Date of publication of application: **06.02.2002**
(21) Application number: 00306606.5
(22) Date of filing: 02.08.2000
(51) Int. Cl.: C11B 3/00, A23D 9/00, A23L 1/30, A61K 31/232

(54) **Purifying crude pufa oils**

(71) Applicant: DSM N.V., 6411 TE Heerlen (NL)
(72) Inventor: Bijl, Hendrik Louis, 3131 ZD Vlaardingen (NL); Wolf, Johannes Hendrik, 2285 KC Rijswijk (NL)
(74) Representative: Wright, Simon Mark

(57) **Abstract**

A process for preparing an oil mixture of an ω6 PUFA (such as ARA) with ω3 PUFA (such as DHA and/or EPA) is described which can be included into edible formulations such as foodstuffs, and in particular infant formulas. A crude ω6 PUFA (e.g. ARA)-containing oil is admixed with a crude ω3 PUFA (e.g. DHA or EPA)- containing oil to produce a crude oil mixture (or blend). This mixture is then purified before addition to a foodstuff. Alternatively, a crude ω6 PUFA-containing oil is treated, and a crude ω3 PUFA-containing oil is also treated, and the two purified oils are then blended prior to deodorising and then added to a foodstuff. The purifying includes acid and/or alkali treatment, bleaching, deodorization, filtering, polishing or cooling. The purifying removes trace metals, pigments, carbohydrates, proteins, sulphur, sterols, mono- or di- glycerides from the oils which renders them suitable for human consumption.

## Description

The present application relates to oil mixtures containing at least two polyunsaturated fatty acids (PUFAs), and processes for their preparation by combining a first PUFA-containing oil with a second PUFA-containing oil (usually a different PUFA from the PUFA contained in the first oil). In particular, the invention relates to preparing purified oil mixtures (blends) by admixing a crude ω6 PUFA (e.g. ARA)-containing oil with a crude ω3 PUFA (e.g. DHA or EPA) containing oil to form a crude oil mixture, and then processing (e.g. purifying ) the oil blend.

It is known to blend PUFA oils. For example, WO-A-92/12711 (Martek Corporation) suggests blending microbial oils and using such a blend in infant formula. The microbial oils specified contain EPA, DHA or ARA, although fish and vegetable oils are also mentioned. The document teaches the blending of the microbial oils to mimic the PUFA content in human breast milk. In particular, it advocates blends of DHA and ARA in ratios of 1-5:2-12, such as at a ratio of about 1:3.

In the Examples of WO-A-92/12711 the fermentation methods yield three different microbially derived oils, ARASCO, DHASCO, and EPASCO, and these are all in crude forms (see Examples 1 to 3). They are then blended and it is suggested that they can be added to infant formula. However, no infant formula (or other foodstuff) is actually prepared, and it has been found that these crude oils are not always suitable for inclusion into infant formula. For example, crude oils can contain various compounds (phospholipids, pigments, trace metals, free fatty acids, mono- and di-glycerides, sterols, sulphur, oxidation products such as aldehydes and epoxides, and various other water or oil insoluble substances) that are undesirable (in an oil blend that is to be incorporated) in (baby) food.

When adding any substance to a foodstuff, care must be taken to eliminate contaminants and other compounds that may have an adverse effect. This is particularly true for substances that are to be introduced into infant formula, because babies and infants are particularly susceptible to impurities or other undesirable substances. It is therefore an aim of the present invention to at least mitigate these disadvantages, and if possible prevent or eliminate them.

The present invention at its broadest concerns the use of one or more purifying techniques to convert the crude oil into a purified oil, the purified oil being suitable for inclusion into an infant formula or other foodstuff. The purifying may remove impurities or other undesirable substances, and as a result is suitable for ingestion by humans.

The first aspect of the present invention therefore relates to a process for preparing an oil mixture, the process comprising:
(a) combining a crude ω6 PUFA (e.g. ARA)-containing oil with a crude ω3 PUFA (e.g. DHA or EPA)-containing oil to produce a crude oil mixture; and
(b) purifying the crude oil mixture to produce a purified ω6 and ω3 PUFA-containing oil mixture.

The invention thus conducts purifying after the crude oils have been combined, that is to say it is the crude oil mixture that is purified. This means that one purifying step may be required. Little further processing may then be necessary. It also means that one does not have two oils that may be in slightly different "oxidative states" (for example if the oxidation levels of the two oils differ slightly).

The process may additionally comprise (for example during purifying) removing one or more of the following components: a phospholipid, trace metal, pigment, carbohydrate, protein, free fatty acid (FFA), oil insoluble substance, water insoluble substance, soap or saponified substance, oxidation product, sulphur, mono- or diglyceride, pigment decomposition product, solvent and/or sterol. The purifying may reduce or remove "off-flavours" and/or improve the stability of the oil.

To effect this the process (e.g. purifying) may comprise degumming (or acid treatment), neutralization (or alkali treatment), water washing, bleaching, filtering, deodorising, polishing and/or cooling (or winterization). Preferably the purifying comprises acid treatment and/or alkali treatment (degumming and neutralisation). Alternatively purifying methods may comprise bleaching and/or deodorization. Preferably however the purifying will involve bleaching and/or deodorization, and optimally in addition acid and alkali treatment. The combining in (a) may thus occur after one or more of these (purifying) steps are performed, in other words some of the steps may be performed before combining in (a), and some may be performed on the (combined) oil mixture in the purifying in (b). In this situation it is preferred that the combining in (a) occurs before deodorization. Steps such as degumming, alkali treatment, bleaching and/or cooling can be performed either on the. separate oils, before combining, or on the oil mixture. Thus a "crude" oil can refer to a non-deodorised oil, while a purified oil can be a deodorised oil. The invention may thus comprise combining an ω6 PUFA-containing oil with an ω3 PUFA-containing oil to form an oil mixture, and then purifying (e.g. deodorising) the oil mixture.

These additional processes are preferably performed during purifying in (b), but one or more of them may be performed either on the ω6 PUFA-containing oil and/or the ω3 PUFA-containing oil before the oils are combined in (a).

A second aspect relates to a purified oil mixture comprising a ω6 PUFA and a ω3 PUFA, which is advantageously suitable for human (e.g. infant) consumption. This can be a purified oil blend which is preparable by a process of the first aspect.

A third aspect relates to an edible formulation comprising a purified oil mixture of the second aspect. This formulation may be a foodstuff, preferably adapted for consumption by humans, and optionally an infant formula, a nutritional supplement or a pharmaceutical composition.

### Purifying processes

### Solvent treatment

This may reduce or remove gums and/or proteins. Polar solvents are preferred, e.g. acetone. This is particularly suitable for ω3 oils, e.g. DHA-containing oils.

### Degumming (or acid treatment)

This may remove or reduce a variety of compounds such as phospholipids, trace metals, pigments, carbohydrates and/or proteins. It is particularly desirable to remove some or all of the (hydratable and non-hydratable) phospholipids. These can be coloured compounds and as such undesirable. They may cause complications in (if applicable) a later alkali treatment step because of their emulsification properties. In addition, they may cause undesirable browning if a deodorization step is employed. Phospholipids can settle out in storage vessels and once this happens they can be difficult to remove.

The process preferably involves the addition of water and phosphoric and/or citric acid (e.g. H₃PO₄) to the oil, followed if necessary by mixing. If citric acid is used this is preferably a 50% aqueous solution. For phosphoric acid an 85% aqueous solution can be employed. Following this the oil may be heated, for example to break up any emulsion that has formed. The "gums" or other unwanted substances may then be removed, for example by centrifugation.

The degumming may first start with heating. If necessary heating may be employed, for example at a temperature of 50 to 80°C, such as 55 to 75°C, optimally from 60 to 70°C.

The acid can then be added. For phosphoric acid, this may be from 0.1 to 2.0g, such as from 0.5 to 1.5g, preferably from 0.8 to 1.2g phosphoric acid per 1kg oil. These figures are based on an 85% phosphoric acid solution and so the amount of acid can vary for different acid concentrations (e.g. 50 to 95%, 70 to 90%, 80 to 88% phosphoric acid) and these (*pro rata*) equivalents are contemplated.

In some processes water may also be added, although this can usually be omitted. If employed, the amount of water added may be from 25 to 125%, such as from 50% to 100%, optimally from 70 to 80%, of the weight of phospholipids thought to be present in the oil. In relation to the oil the amount of water may be from 0.1 to 15%, such as 0.5 to 10%, optimally from 1 to 5% (either by weight or by volume). If water is used then preferably the (phosphoric) acid is added to the oil before the water.

This treatment may take from 5 minutes to an hour, such as from 10 minutes to 30 minutes, preferably from 15 to 20 minutes. The temperature may be from 50 to 110°C, preferably from 80 to 100°C, optimally from 70 to 90°C.

If neutralisation or bleaching is also to take place, either of these steps can be combined with degumming. The phospholipid content of the oil before degumming may be from 2 to 3.5% (by weight).

### Alkali treatment (also known as refining)

This may also be referred to as neutralization, since it involves an alkali, which may neutralise any acid present in the oil. This acid may be present as a result of acid treatment, such as degumming as discussed above.

The alkali treatment is intended to remove or reduce free fatty acids (FFAs), phospholipids, pigments, trace metals, oil insoluble substances and/or water insoluble substances. Preferably this stage removes some or all of the free fatty acids. These can cause problems in foodstuffs because of their foaming characteristics. In addition, they can be toxic.

The alkali treatment preferably converts the free fatty acids (FFAs) into soaps or hydrolyses triglycerides (saponification). This results from reaction of alkali metal ions, especially sodium, with the FFA's. Preferably the concentration of FFAs is reduced to below 0.2%, preferably below 0.1%, optimally less than 0.05% (by weight).

This stage can be a batch or continuous process. Preferably, before the alkali is added, the FFA content is estimated by means of known techniques, in particular acid value. (For example the FFA content can be checked by titration). An excess of alkali may be used, for example if the oil has already been subjected to acid treatment, such as degumming.

If necessary heating may (first) be employed, for example to a temperature of from 50 to 90°C, such as 55 to 85°C, optimally from 60 to 80°C. A titration may then be performed to determine how much acid is present, and therefore how much alkali may be required.

The alkali may then be mixed with the oil. Suitable alkalis are alkali or alkaline earth metal or ammonium hydroxides. Alkali metal hydroxides are preferred as these can minimise interference with a deodorising step if employed later: the favourite is sodium hydroxide. The alkali may be added at a concentration of from 10 to 15%, such as from 12 to 13%. The amount of alkali added may be sufficient to neutralise the oil.

The resulting soaps or saponified substances can then be removed or separated by centrifugation, for example using self-cleaning centrifuges. These may be hermetically sealed or closed to avoid contact with air.

The or any (remaining) soaps may be removed by washing, for example using water, e.g. at a temperature of from 80 to 120°C, preferably 90 to 100°C. A second centrifugation may then be employed. If a water washing stage is additionally employed this may be followed by a drying stage, if appropriate.

An alternative method of removing FFAs, either in addition to or instead of alkali treatment, is to employ distillation or deodorization (sometimes called physical refining in the art), the latter technique being discussed later.

The crude oil before being subjected to this stage (or any FFA removing treatment) may have a FFA concentration of from 0.2 to 0.6%, such as from 0.3 to 0.5%.

### Antioxidants

Any suitable antioxidants can be employed. Examples include tocopherol (e.g. 400 to 1200, preferably 600 to 1000, optimally 600 to 800ppm) and/or ascorbylpalmitate (e.g. 50 to 150, preferably 70 to 130, optimally 80 to 120 mg/kg oil).

### Bleaching

In this stage it is intended to remove or reduce pigments, oxidation products, trace metals, sulphur and any soaps or saponified products (for example resulting from alkali treatment). In particular, this stage removes not only coloured compounds, but pigments such as carotenes (e.g. β-carotene), chlorophylls, browning compounds, compounds that impart flavours, hydroperoxides and/or any undesirable metals.

If drying is employed prior to bleaching this may be at a temperature of from 50 to 100°C, preferably from 60 to 90°C, optimally from 70 to 80°C. The drying may be conducted under a vacuum.

Bleaching preferably comprises adsorption of one or more of these impurities. One may use any suitable refiner or adsorbent (the terms are used interchangeably). This may comprise a finely divided and/or activated substance, for example finely divided natural or activated earth, carbon, and/or bleaching earth (e.g. a bleaching clay or bentonite based product) . The oil is mixed with the chosen adsorbent. The amount of adsorbent used will depend on the colour of the oil and the amount of impurities thought to be in the oil. However, as a guide an amount of 0.25 to 5%, preferably 0.5 to 3%, optimally from 0.75 to 1.5% of adsorbent in relation to the oil (by weight) can be employed.

The oil and adsorbent mixture may then be sprayed, for example under vacuum. This may remove air. The oil then may be heated, for example at a temperature of from 80 to 130°C, preferably 90 to 120°C, or optimally from 100 to 110°C. The contact time between oil and adsorbent may be from 5 to 40 minutes, preferably from 10 to 30 minutes, optimally from 50 to 25 minutes. The oil may then be cooled and/or filtered.

The bleaching may comprise contacting the oil with a soap-removing additive, for example a treated silica product such as Trisyl™ especially if alkali treatment has been performed. This may remove metals and/or gums. It may be added at from 0.1 to 5kg, e.g. 0.5 to 3.0kg, per kg of oil.

This stage can be employed as a batch or continuous process. The bleaching and/or filtering may be conducted without air being present, for example, under a vacuum or using (a blanket of) an inert gas, such as N₂.

### Cooling (or winterization)

This can remove saturated triglycerides (i.e. triglycerides from saturated fatty acids). This is useful because saturated triglycerides can increase the turbidity point.

This stage involves cooling the oil, preferably so that crystals (comprising the compound or impurity to be removed) are formed. Thus, preferably crystals containing saturated triglycerides will be produced. The oil may be stored in a tank, and if conditions permit, this may be an outside tank. Storage may take place during cooler conditions, for example during winter. Alternatively the oil can be cooled, e.g. using heat exchangers.

Preferably relatively large crystals form. Indeed, it is advantageous that large crystals form or crystals that have a greater density than the oil. Thus the crystals preferably fall or migrate to the bottom of the oil, for example to form a sediment.

Preferably the oil is cooled slowly. The final (or cooled) temperature is preferably from 0 to 10°C, such as from 3 to 7°C, optimally from 5 to 6°C. During cooling the oil may be agitated, but preferably only under mild agitation conditions. Advantageously high shear rates are avoided. The time taken to cool the oil to this temperature may be from 12 to 36 hours, such as from 18 to 30 hours, optimally from 21 to 27 hours.

The oil may then be filtered. This is so that any sediment (or crystals) can be removed. This may involve standard equipment such as a plate and frame filterpress. Alternative methods of removing the sediment or crystals include centrifugation or vacuum filtration.

### Deodorization

This may remove or reduce fatty acids and free fatty acids, mono- and diglycerides, oxidation products, pigment decomposition products, solvents and/or sterols. In particular, it can remove unwanted flavouring compounds, for example aldehydes and ketones. It may also remove hydrocarbons, for example resulting from the breakdown of hydroperoxides. Other compounds that may be removed include sterols and tocopherols.

Preferably deodorization comprises distillation, such as with steam. It is preferably conducted under a vacuum, or at least reduced pressure (e.g. 1 to 8, such as 2 to 4 mbar). The temperature of the oil may be from 100 to 300°C, such as from 150 to 250°C, optimally from 180 to 220°C.

This stage may be performed as a batch, semi-continuous or a continuous process. Preferably FFA levels are reduced in the oil to below 0.06%, preferably below 0.04%, optimally below 0.03% (for example using the POV (peroxide value) and AnV (anisidine value) parameters).

### Polishing

This may remove some or all of the (last traces of) oil insoluble substances. It may comprise clarifying the oil, for example using a candle or a (cartridge) filter.

### Preferred Process

Preferably the (purifying ) process of the invention thus comprises:
(a) combining a (crude) ω6 PUFA-containing oil with a (crude) ω3 PUFA-containing oil to produce a (crude) oil mixture. This step can be performed now (or first), or later after one or more of steps (c) to (i) have been performed. However, combining should take place before step (j);
(b) purifying, which can be performed either on the separate ω6 and ω3 PUFA-containing oils or on the oil mixture (resulting from combining). The purifying can thus comprise;
(c) optionally, treatment with an acid or degumming, or removing phospholipids, trace metals, pigments, carbohydrates and/or proteins;
(d) optionally, treatment with an alkali or removing free fatty acids, phospholipids, pigments, trace metals, water-insoluble substances and/or oil-insoluble substances;
(e) optionally, washing with water and if necessary, drying:
(f) optionally, adding one or more antioxidants;
(g) optionally, bleaching or removing pigments, oxidation products, trace metals, sulphur and/or soap;
(h) optionally, filtering;
(i) cooling or winterising or removing saturated triglycerides. If the ω6 and ω3 PUFA-containing oils have not yet been combined, then they are combined (as in (a)) to form an oil mixture at this stage. Up to this point the oils may still be regarded as being crude;
(j) deodorising or removing free fatty acids, mono- and di- glycerides, oxidation products, pigment decomposition products, solvents and/or sterols;
(k) optionally, polishing or removing oil-insoluble substances.

In some purifying processes steps (c), (d) and (g) are not optional. In other words, although above only the deodorising step (j) may be performed, in some embodiments steps (c), (d) and (g) can be included. Alternatively or in addition step (i) is not optional, and/or preferably one or both of steps (f) and (k) are not optional.

The above steps (c) to (i) are applicable to any purifying protocol. For example this may be conducted on the crude oil mixture. However one or more of steps (c) to (i) above can be performed on the crude ω6 PUFA-containing oil and/or on the ω3 PUFA-containing crude oil, the oils can then be combined and steps (j) and/or (k) performed on the oil mixture.

After purifying (and so in a purified oil), the content of (undesirable or heavy) metals is preferably significantly reduced. For arsenic (As) the amount is preferably below 0.2 ppm, preferably below 0.1 ppm, optimally below 0.05 ppm. For lead (Pb) the amount is preferably below 0.06ppm, preferably below 0.04ppm, optimally below 0.02ppm. The preferred amounts of mercury (Hg) and cadmium (Cd) are the same as those specified for lead.

### PUFAs and oils

Preferred PUFAs are C18, C20 or C22 (ω6 or ω3) PUFAs. Preferred PUFAs include:
(ω3) docosahexaenoic acid (DHA), suitably from algae or fungi, such as the dinoflagellate *Crypthecodinium* or the fungus *Thraustochytrium*;
(ω6) γ-linolenic acid (GLA);
(ω3) α-linolenic acid (ALA);
(ω6) dihomo-γ-linolenic acid (DGLA);
(ω6) arachidonic acid (ARA); and
(ω3) eicosapentaenoic acid (EPA).

The ω3 PUFA (e.g. DHA)-containing oil may be a marine, e.g. fish (such as tuna) oil. The ω6 and/or ω3 PUFA (e.g. ARA, DHA or EPA)-containing oil can be a microbial or single cell oil.

Preferably both ω6 and ω3 PUFAs (e.g. GLA, ARA and EPA) can be obtained from fungi, such as *Mortierella, Pythium* or *Entomophthora*. PUFAs of ω3 (e.g. EPA) can be produced from algae such as *Porphyridium* or *Nitzschia*.

Preferably the ω6 or ω3 (e.g. ARA, DHA or EPA containing) oil is a microbial oil, produced by a microorganism. This may be a bacteria, yeast, algae or fungi. Preferred fungi are of the order *Mucorales*. The fungus may be of the genus *Mortierella*, *Phycomyces, Blakeslea* or *Aspergillus*. Preferred fungi are of the species *Mortierella alpina*, *Blakeslea trispora* and *Aspergillus terreus*.

Preferred yeasts are of the genus *Pichia* or *Saccharomyces*, for example *Pichia ciferrii*. Bacteria can be of the genus *Propionibacterium*. Preferred algae are dinoflagellate and/or belong to the genus *Crypthecodinium,* for example are of the species *Crypthecodinium cohnii*.

The ω6 and/or ω3 PUFA-containing oil may be an edible oil or a vegetable oil. These include blackcurrant, borage and primrose oils, and often contain an ω6 PUFA, e.g. GLA. They also include olive, sunflower and soybean, soy flower oils, for example cooking and/or salad oils.

### PUFA oil production

In the process of the invention the microorganism is suitably first fermented, such as in a fermenter vessel containing a culture medium. The fermentation conditions may be optimised for a high PUFA content in the resulting biomass (and, later, in the oil). If desirable, and for example after fermentation is finished, the microorganisms may be killed or pasteurised. This may be to inactivate any undesirable enzymes, for example enzymes that might degrade or reduce the yield of the PUFAs.

The biomass may then be removed from the fermenter, and if necessary liquid (usually water) removed therefrom. Any suitable solid liquid separation technique can be used. This (dewatering) may be by a mechanical method such as centrifugation and/or filtration. Suitable centrifuges can be obtained from Westfalia™ or Tetra Laval™. Centrifugation may last for from 2 to 8, such as from 3 to 7, optimally from 4 to 6, minutes. Residence times are from 0.1 to 3, such as 0.3 to 2, optimally 0.5 to 1.0, minutes. The centrifuge may operate at from 2,000 to 8,000g, such as from 3,000 to 7,000g, optimally from 4,000 to 6,000g. Following disruption and separation the process of the invention may further comprise extracting, purifying or isolating one of more PUFAs.

The cells may then be washed, for example using an aqueous solution (such as water) for example to remove any extracellular water soluble compounds.

Preferably the crude ω6 PUFA (e.g. ARA)-containing oil comprises from 15 to 25% of the ω6 PUFA. The purified ω6 (e.g. ARA) oil preferably comprises from 35 to 45% of the ω6 PUFA. The same figures may apply to ω3 crude and/or purified oils.

### Production of Crude Oils

The crude oil may be a microbial (e.g. single cell) crude oil, or it may be a marine (e.g. fish) oil or vegetable oil (either crude or partially treated). In particular, crude oils containing ω3 PUFAs (DHA and/or EPA) can be marine oils. If the PUFA oil is to contain GLA, then the crude oil may be a vegetable oil, for example blackcurrant, borage, sunflower, soybean or primrose oil.

A number of documents describe the production of crude PUFA oils. Microbial oils containing ARA are disclosed in WO-A-92/13086 (Martek), EPA in WO-A-91/14427(Martek) and DHA in WO-A-91/11918 (Martek). The present Applicant has already described various methods for extracting PUFA oils from microbial sources, and these can be found in WO-A-97/36996 and WO-A-97/37032 (both Gist-brocades). Preparation of ARA, DHA and EPA-containing oils is also described in WO-A-92/12711 (Martek).

In the oil, it is preferred that most of the PUFA is in the form of triglycerides. Thus, preferably at least 50%, such as at least 60%, or optimally at least 70%, of the PUFA is in triglyceride form. Of these triglycerides, preferably at least 40%, such as at least 50%, and optimally at least 60% of the PUFA is present at the α-position of the glycerol (in the triglyceride backbone), also known as the 1 or 3 position. It may be preferred that at least 20%, such as at least 30%, optimally at least 40% of the PUFA is at the β(2) position.

Suitably the purified oil will solidify at a temperature of from 4 to 6°C. In the purified oil the triglyceride content is preferably at least 90%, such as at least 93%, and optimally at least 95%. The mono-glyceride and/or di-glyceride content is preferably less than 7%, such as less than 5%, optimally less than 3%. The amount of monoglycerides may be less than 0.5%, such as less than 0.1%, optimally less than 0.1%. The content of sterol in the purified oil is preferably less than 5%, such as less than 3%, optimally less then 2%.

In the purified oil, the content of the (desired) PUFA is preferably at least 30%, such as at least 35%, optimally at least 40%.

The amount of free fatty acids is preferably no more than 0.5%.

### Mixtures (blends)

In this specification the term "mixture" includes a combination of the oils (for example where the oils, after being combined, can be separated back into their original component oils) and blends (where once combined the oils are inseparable). An example of the former is where one (or even both) of the oils are (e.g. micro-) encapsulated so that although the oils are mixed, they can be separated into the original oils before mixing. However blends are preferred, where the oils are intimately mixed and separation into the original constituent oils is impossible.

The ratio of ω6:ω3 PUFAs in the purified oil mixture is preferably from 1:5 to 5:1, optimally from 1:1 to 1:2. Preferably the amount of ω6 PUFA (especially if it is EPA) is at least 1/5^{th} of the amount of the ω6 PUFA (especially if it is ARA) in the purified oil mixture. Indeed, it is preferred that the ω3 PUFA is at least 25%, such as at least 30%, optimally 35% of the ω6 PUFA content.

The invention will now be described, by way of example, with reference to the following Examples, which are not to be construed as being limiting, but instead are merely illustrative of the present invention.

### Example 1

Crude ARA-containing oil was obtained using the method of Example 16 of the WO-A-97/36996 (Gist-brocades, 2777), using hexane as the solvent for extraction. The ARA content was 35%. Crude DHA oil (27% DHA) was obtained from a marine source (tuna oil) from PronovaBiocare A.S., P.O. Box 2109, N-3202 Sandefjord, Norway (EPAX™ 0525TG).

The ARA- and DHA-containing oils were then mixed together to form a blend. A sterile stainless steel tank was first charged with 10kg of the crude ARA. To the tank was added 30kg of crude DHA oil, with stirring, over a time of 15 minutes (excluding air). This gave a crude oil mixture with an ARA:DHA oil (weight) ratio of 1:3 (ARA:DHA ratio of 1:2.31).

The crude oil mixture was then purified according to the following protocol.

### Purifying

This consisted of four principal steps, with intermediate steps as and when necessary. The four main stages were:
acid treatment (degumming);
alkali treatment (neutralisation);
(if necessary water washing and drying, for example to remove soaps or soap residues);
bleaching;
(if necessary) filtration, for example to remove adsorbents used in bleaching;
deodorising;
(if necessary) polishing; and
(if necessary cooling or winterization).

1. Degumming (acid treatment). This was performed primarily to remove phospholipids. The oil was heated to 70°C. Phosphoric acid was dissolved in water to a concentration of 85%. This was added to the oil (about 3% of the weight of phospholipids in the oil) at 1.5g phosphoric acid solution per kg oil. The oil was kept at a temperature of 80°C for 15 minutes. The mixture of oil and phosphoric acid initially formed an emulsion, which was demulsified on further heating. The gums were then removed by centrifugation.
2. Alkali purifying (neutralisation). A titration was performed to determine the amount of acid present in the oil, and hence to calculate the amount of alkali required for neutralisation. To the oil alkali (sodium hydroxide, 15%) was added to the oil and well mixed. The resulting soap (saponified substances) were then separated by using self-cleaning centrifuges (hermetically closed to avoid contact with air). Any traces of soap were then washed out by using hot water (94°C) followed by centrifugation.
3. Bleaching. The oil was first dried at 70°C under a vacuum. To the oil was then added an adsorbent (Trisyl™, Grace Davison, 1% based on the volume of the oil) and mixed in. The oil was heated to 105°C and the oil adsorbent mixture sprayed into an agitated bleaching tank to remove air. The adsorbent was allowed to stay in contact with the oil for about 20 minutes, whereupon the oil was allowed to cool and filtered.
4. Winterization (cooling). The oil (now at about 35°C) was slowly cooled to 4°C under mild agitation conditions. This took about 24 hours. The oil was kept at 4°C for another 24 hours. The oil was then filtered using a plate and frame filter press at a pressure of 200kg/m² (0,2 to 0.5 bar).
5. Deodorization. The oil was steam distilled under vacuum at a temperature of 180°C and low pressure (2 to 4 mbar).

A comparison of analyses of the crude ARA before purifying and the purified and blended ARA and DHA-containing oil are provided in Table 1.

**Table 1**

| Substance and amount | ARA Crude Oil (before purifying ) | Blended Oil (after purifying ) |
|---|---|---|
| Phospholipids (%) | 2 - 3.5 | 0.05 |
| FFAs (%) | 0.4 | <0.05 |
| Phosphatide (P/ppm) | 50-100 | <10 |
| Fe (ppm) | 0.5-2.0 | <0.05 |
| Cu (ppm) | 0.07 | <0.02 |
| ARA(g/kg) | 350 | 85.5 |
| ARA (as % of total fatty acids) | 38 | 9.5 |
| Water (%) | 0.5 | 0.1 |
| Peroxide value (POV) | 5-10 | <1.0 |

### Example 2

A crude ARA-containing oil was prepared according to the method of Comparative Example 1 as described in WO-A-97/43362 (Gist-brocades, 2798). This contained approximately 30% ARA.

A DHA crude oil was obtained using the protocol described in Example 21 of WO-A-97/36996 (Gist-brocades, 2777). This oil contained 60% triglyceride, 12% diglycerides and 3.7% sterols. The DHA concentration was 32.6%.

Each of the crude oils were then treated using the same protocol as Example 1, with the following variations:
(i) in the acid treatment, the contact time with phosphoric acid was 20 minutes and the oil temperature was 70°C for the ARA oil, and for the DHA oil citric acid (50%) was used instead (20 minutes, 75°C);
(ii) in the alkali treatment the concentration of sodium hydroxide was 13%, and the hot water treatment (water washing) was omitted;
(iii) bleaching used 1.5% of adsorbent Tonsil™ (Süd-Chemie, Munich, Germany) and was at 100°C for 15 minutes.

Ten kilogrammes of each of the two resulting crude oils were blended in a 30 litre tank with mixing, to form an ARA:DHA (by volume) blend of 1:1 (ARA:DHA ratio of 1:1.09). The blend of the two oils was then deodorised at a temperature of 200°C and then clarified using a cartridge filter.

### Example 3

A purified ARA microbial oil (38% ARA) derived from *Mortierella alpina*, was obtained (5kg) from DSM N.V., Food Specialties Division, P.O. Box 1, 2600 MA Delft, The Netherlands under the trademark OPTIMAR™. This oil had been purified using the protocol of Example 1. A purified DHA-containing oil (10kg) from Pronova, Norway, containing 27% DHA (EPAX™ 0525TG) was then blended with the purified ARA oil. The two oils were mixed with each other in a stainless steel vat equipped with a stirrer (ARA:DHA ratio of 1:1.54).

### Example 4

The crude ARA oil employed in Example 1 was purified using the protocol of Example 1, with the following variations:
(i) phosphoric acid (80%) was added at 2% of the volume of the oil;
(ii) the alkali was KOH (10%);
(iii) water washing was at 85°C but with a small volume of water so no subsequent washing was required;
(iv) bleaching was at 90°C for 30 minutes; and
(v) cooling was down to 5°C and kept at 5°C for 2 days.

Table 2 gives the analyses of the oil before and after purifying .

**Table 2**

| Substance and amount | Crude Oil (before purifying ) | Purified Oil |
|---|---|---|
| Phospholipids (%) | 2 - 3.5 | 0.05 |
| FFA's (%) | 0.4 | <0.05 |
| Phosphatide (P/ppm) | 60-100 | <8 |
| Fe (ppm) | 1.0 | <0.05 |
| Cu (ppm) | 0.06 | <0.02 |
| ARA (g/kg) | 350 | 175 |
| ARA (%) | 38 | 19 |
| Water (%) | 0.5 | 0.1 |
| Peroxide value (POV) | 7 | <1.0 |

The crude DHA oil from Example 2 was then also subjected, separately and independently, to the same protocol as the crude ARA oil. The resulting ARA oil (10kg) was then blended with the resulting DHA oil (33% DHA, 15kg) together in a stainless steel vat equipped with a stirrer. The blended oil had an ARA:DHA ratio of 1:1.16 and was then subjected to deodorization at 220°C.

A review of the various purifying protocols is shown in Table 3.

**Table 3**

| Purifying Step | Example No. | | |
|---|---|---|---|
| | 1 | 2 | 4 |
| Acid Treatment | 1.5%, 80°C, 15mins | 1.5%, 70°C, 20mins | 2%, 80°C, 15mins |
| Alkali treatment | 15% NaOH | 13% NaOH | 10% KOH |
| Water washing | 94°C | 94°C | 85°C |
| Drying | 70°C, vacuum | 70°C vacuum | No |
| Bleaching | 1% adsorbent 105°C, 20mins | 1.5% adsorbent, 110°C, 15mins | 1% adsorbent, 90°C, 30mins |
| Filtering | Yes | Yes | Yes |
| Cooling | Cool to 4°C over 24 hrs, keep at 4°C for 24 hrs | As 1 | Cool to 5°C over 12 hrs, keep at 5°C for 2 days |
| Polishing | No | Yes | No |
| Deodorising | 180°C | 200°C | 220°C |
| ω6 PUFA | ARA (35%) | ARA (30%) | ARA (38%) |
| ω3 PUFA | DHA (20%) | DHA (32.6%) | DHA (33%) |
| ω6: ω3 ratio | 1:2.31 | 1:1.09 | 1:1.54 |

Example 3 uses the same purifying as Example 1, except uses crude ARA (38%) and DHA (25%) oils (ARA:DHA ratio 1:2.6).

### Example 5

The protocol of Example 1 was repeated except that crude EPA containing oil (obtained by using the Example of WO-A-91/14427 to obtain an extracted single cell oil, Martek) was used instead of crude DHA oil. The ARA oil (35% ARA, 10kg) was then blended with the EPA oil (37% EPA, 5kg) together in a stainless steel vat equipped with a stirrer. The resulting purified and blended oil had an ARA:EPA ratio of 1:0.53.

### Examples 6 to 8

The oil blend of Example 1 (0.5kg) was mixed with lactose particles (10kg) in a Lödige mixer by adding the oil blend at a rate of 10 kilograms per minute. The resulting powder was stored under nitrogen. The powdered mixture was then added to a powdered infant formula (20kg of SMA Gold™, United Kingdom). The same procedure was conducted using the purified ARA/DHA blend of Example 4 and the purified ARA/EPA blend of Example 5.

### Example 9

A batch of 200 kg of powdered infant formula containing 2kg of a DHA/ARA oil blend as a homogeneous liquid was prepared according to the following protocol. To the infant formula the oil blend was added to a temperature of 50°C. The blend was sprayed at a rate of 0.5 kilograms per hour onto infant formula moving through a fluidised bed at a rate of 50 kilograms per hour. The temperature was set so that the water loss from drying was between 1 and 2%.

### Example 10

A batch of 1,000kg of infant formula was supplemented with an oil blend according to Example 2 as follows. A premix was first prepared containing 27kg of the ARA/DHA oil blend. This premix also contained vitamins and minerals and lactose, and had a total weight of 200kg. The premix was then added to 800kg of spray dried infant formula, and then mixed in a Chronicle Nauta 50 RK mixer. This powdered instant formula was made into milk for consumption by babies by adding sterile water (13g of powdered infant formula to 90ml water).

## Claims

1. A process for preparing an oil mixture, the process comprising:
(a) combining a crude ω6 PUFA-containing oil with a crude ω3 PUFA-containing oil to produce a crude oil mixture; and
(b) purifying the crude oil mixture to produce a purified ω6 and ω3 PUFA-containing oil mixture.

2. A process according to claim 1 wherein:
(i) the purifying comprises removing phospholipids, trace metals, pigments, soaps, carbohydrates, proteins, sulphur, sterols, mono- or diglycerides, saturated triglycerides and/or free fatty acids;
(ii) the oil mixture is an oil blend; and/or
(iii) the ω6 PUFA is ARA and/or the ω3 PUFA is DHA or EPA.

3. A process according to claim 1 to 2 wherein the purifying comprises acid treatment, alkali treatment, neutralisation, water washing, filtering, polishing, winterization, bleaching and/or deodorization.

4. A process according to any preceding claims which additionally comprises:
(c) optionally, treatment with an acid or degumming, or removing phospholipids, trace metals, pigments, carbohydrates and/or proteins;
(d) optionally, treatment with an alkali or removing free fatty acids, phospholipids, pigments, trace metals, water-insoluble substances and/or oil-insoluble substances;
(e) optionally, washing with water, and if necessary drying;
(f) adding one or more antioxidants;
(g) optionally, washing with water, bleaching or removing pigments, oxidation products, trace metals, sulphur and/or soap;
(h) optionally, filtering;
(i) optionally, cooling or winterising or removing saturated triglycerides;
(j) deodorising or removing free fatty acids, mono- or di- glycerides, oxidation products, pigment decomposition products, solvents and/or sterols;
(k) optionally, polishing or removing oil-insoluble substances.

5. A process according to any preceding claims wherein:
(i) the crude ω6 PUFA (e.g. ARA)-containing oil contains from 15 to 25% of the ω6 PUFA;
(ii) the purified ω6 PUFA oil contains from 35 to 45% of the ω6 PUFA;
(iii) one or more of (c), (d) and/or (i) of claim 4 are not optional; and/or
(iv) one or more of (c) to (i) of claim 4 are performed on the crude ω6 PUFA-containing oil and/or the crude ω3 PUFA-containing oil, the resulting oils are then combined as in (a), and the oil mixture is then treated according to (j) and/or (k).

6. A process according to any preceding claim wherein either:
(i) the ω3 PUFA (e.g. DHA)-containing oil is a fish oil and/or the ω6 or ω3 PUFA (e.g. ARA, DHA or EPA)-containing oil is a microbial oil; and/or
(ii) the crude ω6 PUFA-containing oil is derived from a fungus of the order *Mortierella*, optionally from the fungus *Mortierella alpina*, and/or the ω3 PUFA-containing oil is derived from the organism *Crypthecodinium cohnii*; or
(iii) either the ω6 PUFA or ω3 PUFA-containing oil is a vegetable oil.

7. A process according to any preceding claim wherein in the purified oil mixture:
(i) the amount of ω3 PUFA (e.g. EPA) is at least 20% of the amount of the ω6 PUFA (e.g. ARA); and/or
(ii) the purified oil has an ω6:ω3 (e.g. ARA:DHA) ratio of from 1:5 to 5:1, optionally of from 1:1 to 1:2.

8. A process according to any preceding claim wherein the purified oil has at most 0.1% free fatty acids, at most 0.2% of phospholipids, a phosphorus content below 10ppm, an iron content below 0.05ppm, a copper content below 0.02ppm and/or a peroxide value (POV) below 1.0.

9. A purified oil mixture comprising a ω6 PUFA and a ω3 PUFA, which is suitable for human consumption, and optionally is a purified oil blend which is preparable by a process according to any preceding claim.

10. An edible formulation comprising a purified oil mixture according to claim 9, for example a foodstuff (preferably adapted for consumption by humans and optionally an infant formula) a nutritional supplement or a pharmaceutical composition.
